# EUROPEAN PATENT APPLICATION

(11) **EP 3 879 853 A1**
(43) Date of publication of application: **15.09.2021**
(21) Application number: 20162605.8
(22) Date of filing: 12.03.2020
(51) Int. Cl.: H04R 25/00, A61B 5/00

(54) **ADJUSTING A HEARING DEVICE BASED ON A STRESS LEVEL OF A USER**

(71) Applicant: Sonova AG, 8712 Stäfa (CH)
(72) Inventor: STROPAHL, Maren, 8032 Zürich (CH); LEMKE-KALIS, Ulrike, 8053 Zürich (CH); LAUNER, Stefan, 8053 Zürich (CH); ROECK, Hans-Ueli, 8634 Hombrechtikon (CH)
(74) Representative: Qip Patentanwälte Dr. Kuehn & Partner mbB

(57) **Abstract**

The invention relates to a method (300) for adjusting a hearing device (102). The hearing device (102) comprises a microphone (111) for generating audio data (204), a computer (128) with a sound processor (112) for processing the audio data (204) and a sound output device (114) for outputting the processed audio data (218) to a user of the hearing device (102). The method (300) comprises: receiving (310) sensor data (200) at the computer (128) from a sensor (121) adapted for measuring at least one physiological indicator for a stress response of the user; receiving (310) audio data (204) at the computer (128) from the microphone (111); determining (320) at least one stress situation classification value (206) with respect to a specific stress situation of the user with a stress classifier (208) based on the sensor data (200); when the stress classifier (208) determines at least one specific stress situation classification value (206): starting (330) a sound program (212) comprising sound processing parameters (214) associated with the at least one specific stress situation classification value (206); processing (340) the audio data (204) with the sound processor (112), wherein the sound processing parameters (214) are applied to the sound processor (112) when the sound program (212) is started; and outputting (350) the processed audio data (218) with the sound output device (114) to the user.

## Description

### FIELD OF THE INVENTION

The invention relates to a method, a computer program and a computer-readable medium for adjusting a hearing device. Furthermore, the invention relates to a hearing device for carrying out the method and a hearing system with such a hearing device and a mobile device.

### BACKGROUND OF THE INVENTION

Hearing devices are generally small and complex devices. They may include a processor, microphone, speaker, memory, housing and other electronic and mechanical components. Some examples of hearing devices are Behind-The-Ear (BTE), Receiver-In-Canal (RIC), In-The-Ear (ITE), Completely-In-Canal (CIC) and Invisible-In-The-Canal (IIC) devices. A user may prefer one of these hearing devices over another based on hearing loss, aesthetic preferences, lifestyle needs and budget.

It is well established that cognitive load increases in acoustically difficult situations. In such stressful situations, a cognitive effort required by a user of a hearing device to be able to follow and participate in a conversation is greater than usual, which may lead to faster fatigue. Such an increase in cognitive load usually reflects in changes in psychophysiological or vital signs, also called biomarkers, such as, for example, heart rate, heart rate variability, EEG signals or voice characteristics.

Thus, it would be helpful if the hearing device could automatically adapt to a current situation of the user, in particular to a situation where the user is challenged by an increased cognitive load.

### DESCRIPTION OF THE INVENTION

It is an objective of the invention to modify an audio output of a hearing device dependent on a stress situation a user of the hearing device is currently in.

This objective is achieved by the subject-matter of the independent claims. Further exemplary embodiments are evident from the dependent claims and the following description.

A first aspect of the invention relates to a method for adjusting a hearing device. The hearing device comprises a microphone for generating audio data, a computer with a sound processor for processing the audio data and a sound output device for outputting the processed audio data to a user of the hearing device. The hearing device may be part of a hearing system, which additionally may comprise a mobile device, such as a smartphone, smartwatch, tablet computer, etc. The hearing device may be a hearing aid for compensating a hearing loss of the user. The hearing device may be worn by the user, for example, behind the ear or in the ear. The hearing device may also be seen as a pair of hearing devices with one hearing device for each ear of the user. The hearing device may comprise one or two hearing aids and/or a cochlear implant.

According to an embodiment of the invention, the method comprises: receiving sensor data at the computer from a sensor adapted for measuring at least one physiological indicator for a stress response of the user; receiving audio data at the computer from the microphone. A stress response may be a physiological reaction of the user to a stressful situation. The sensor data may be generated by one or more sensors placed in or around an ear of the user, for example in an ear channel. The sensor may be part of the hearing device. It is also possible that the sensor is implemented in a mobile device carried by the user and connected to the hearing device over a wireless data connection. The sensor may comprise at least one of a photoplethysmography sensor, an electrocardiography sensor, an electroencephalography sensor, an electroglottography sensor, a skin conductance sensor and a movement sensor. A movement sensor may for example be an accelerometer, a gyroscope or a magnetometer. The audio data may be generated in the hearing device by digitizing a sound signal with the microphone of the hearing device. It is also possible that the hearing device receives the audio data, e.g. a music stream or voice stream, over a wireless data connection, for example, from the Internet, other hearing devices or a mobile device such as a smartphone, smartwatch, tablet computer, etc.

According to an embodiment of the invention, the method further comprises: determining at least one stress situation classification value with respect to a specific stress situation of the user with a stress classifier based on the sensor data. The stress classifier may be a computer program or computer program module run in the computer of the hearing device and adapted for discriminating between stressful and non-stressful situations of the user or between different kinds of stressful situations of the user. The stress classifier may output one or more stress situation classification values dependent on the sensor data and, additionally, the audio data. Additionally, the audio data may be input into the stress classifier. For example, the audio data may be used by the stress classifier to determine at least one of a sound level, a signal-to-noise ratio, voice characteristics of a voice of the user, an environmental classification and whether the user is listening to media content, such as music, a movie, an audio book, etc., or not. Voice characteristics may be, for example, a frequency, speech rate, respiration rate, roughness, stuttering or trembling of the user's voice. Examples for stress situations, which may be classified in such a way, are physical activities, sustained (high) workload, emotional agitation, exposure to noises, (high) cognitive load, etc. For each such stress situation, a stress situation classification value may be output, such as 0 and 1 or a percentage value for the probability of such a stress situation. In particular, the stress situation classification value may encode a certain amount of cognitive load the user is experiencing.

The stress classifier may have several subcomponents, which, for example, classify different parts of the sensor data, for example photoplethysmography data, electrocardiography data, electroencephalography data, electroglottography data or acceleration data, into several stress situation classification values, which are then processed by a further subcomponent deciding whether a specific stress situation is present or not. The further subcomponent may be based on rules and/or comparators with threshold values.

According to an embodiment of the invention, the method further comprises: when the stress classifier determines at least one specific stress situation classification value: starting a sound program comprising sound processing parameters associated with the at least one specific stress situation classification value; processing the audio data with the sound processor, wherein the sound processing parameters are applied to the sound processor when the sound program is started; outputting the processed audio data with the sound output device to the user.

For example, the sound program may comprise stress situation definition data defining specific ranges of stress situation classification values. The sound program may be started when the stress situation classification values determined by the stress classifier are within the specific ranges.

In the hearing device, several sound programs may be stored, which may be activated and deactivated dependent on the determined stress situation classification values. Every sound program may define when it should be activated and started. For example, there may be at least one sound program to be activated in a normal situation of the user and at least one other sound program to be activated in a stress situation of the user. There may also be a plurality of sound programs to be activated in different kinds of stress situations. Furthermore, each sound program may define settings to be applied to the sound processor of the hearing device when the sound program is started.

The sound processing parameters may define how the audio data are processed, for example frequencies to be amplified, attenuated or shifted and/or frequency bands to be compressed. The sound processing parameters may also define how and which kind of noise is suppressed and/or whether sound from specific directions is amplified or not (also called beam forming). In general, the sound parameters, when applied to the sound processor, may result in an improvement of intelligibility and ease of listening.

It may be that more than one sound program is running at the same time. For example, the audio data may be processed firstly by one sound program and secondly by another sound program.

With this method, sensor data related to high physiological stress of the user can be used to reliably detect an acoustically difficult (communication) situation. In particular, the method makes it possible to distinguish stress situations with high cognitive load, and, therefore, highly relevant for assessing a hearing situation of the user, from less relevant kinds of stress situations, such as emotional discussions, physical activities, active listening to an exciting audio book, watching a horror movie, etc.

According to an embodiment of the invention, the computer comprises a plurality of stress classifiers, each of which outputting at least one stress situation classification value with respect to a specific stress situation of the user. The sound program comprises sound processing parameters associated with a specific set of stress situation classification values. The sound program is started when the stress classifiers determine the specific set of stress situation classification values. A set of stress situation classification values may be seen as a multidimensional output vector of the stress classifiers. Accordingly, the sound program may comprise definition data defining ranges for each component of the output vector, within which the sound program is started.

According to an embodiment of the invention, at least one of the stress classifiers is a sound classifier for additionally classifying the audio data. Each sound classifier may be a computer program run in the computer of the hearing device. The audio data may be analyzed by each sound classifier in a specific way. For example, the sound classifier may evaluate a volume, a signal-to-noise ratio, frequencies, a sound direction, etc. as stress situation classification inputs. It is also possible that the sound classifier uses properties of the audio data, such as speech in noise, music, etc., as stress situation classification inputs. The sound classifier may process the audio data generated by the microphone of the hearing device and/or audio data received from the mobile device, for example during a telephone call.

According to an embodiment of the invention, further stress classifiers may be based on detecting relevant physical activity as source of physiological stress through e.g. evaluating acceleration sensor data and/or heart rate sensor data and/or skin conductance data. Additionally or alternatively, further stress classifiers may be based on detecting relevant emotions such as excitement or fear through vocal analysis, e.g. by evaluating at least one of a sound level, a timbre, a pitch frequency and its variability, a speaking rate, a roughness of articulation and a breathing rate.

For example, the further stress classifiers may evaluate a long term stress, e.g. due to a high working load, by evaluating statistics of a heart rate variability and its recovery at off-work hours, for example at the weekend.

Further sources of physiological stress may be determined based on said sensor data or further sensor data or activity data.

According to an embodiment of the invention, the sensor data comprise at least one of heart activity data acquired with a heart activity sensor, brain activity data acquired with a brain activity sensor, voice activity data acquired with a voice activity sensor and acceleration data acquired with an acceleration sensor. The stress classifier determines the at least one stress situation classification value by classifying at least one of the heart activity data, the brain activity data, the voice activity data and the acceleration data. The heart activity data may comprise at least one of a heart rate and a heart rate variability, i.e. a beat-to-beat timing variation of a heartbeat. The brain activity data may comprise electroencephalography signals such as alpha, beta, gamma, delta or theta wave signals. The voice activity data may comprise at least one of an electroglottography signal, a vocal emotion signal and a speaking speed signal.

According to an embodiment of the invention, the method further comprises determining at least one reference value by analyzing sensor data generated by the sensor for a predefined period of time and/or when the user is in a specific situation, wherein the stress classifier determines the at least one stress situation classification value by evaluating current activity data in relation with the at least one reference value. The sensor data may be generated during a sufficiently long period of time to determine long-term statistics with regard to different physiological stress indicators, which may then be used to classify the sensor data accordingly. For example, the sensor data used for determining the reference value may be generated for at least one hour, at least one day, at least one week or at least one month. A situation in which the reference value is determined may be when the user is in a rested or calm state or in a conversation which is perceived by the user as causing average or less than average cognitive load.

According to an embodiment of the invention, the stress classifier is based on a machine learning algorithm. For example, an artificial neuronal network trained for classifying stress situations based on the sensor data and, optionally, the audio data may be used as a stress classifier.

According to an embodiment of the invention, the sound program comprises sound processing parameters for adjusting at least one of a beamformer and a noise filter of the sound processor. The sound processor may be adapted for amplifying, shifting and/or compressing specific frequencies or frequency bands of the audio data in dependency of the applied sound processing parameters. Such processing may be used for improving the intelligibility and/or comfort of sound signals to be output to the user's ear and is supposed to reduce the physiological stress response of the body.

According to an embodiment of the invention, the hearing device comprises a plurality of sound programs, each of which comprising sound processing parameters associated with at least one specific stress situation classification value. In other words, in each of the different stress situations, the audio data may be processed in a specific way.

According to an embodiment of the invention, the hearing device uses a suitable sound program to improve intelligibility and/or comfort when a physiological stress response by the body is determined, which might be due to an acoustic stress, but not when the physiological stress response is most probably only due to another reason such as physical activity, excitement, fear, etc.

Exemplary, the following rules may be applied to activate a sound program:
when physiological stress and at least one of high sound levels or a bad signal-to-noise ratio are detected: starting a stress reducing sound program; and/or
when at least one of high sound levels or a bad signal-to-noise ratio, but no short-term physiological stress is detected: starting another sound program with less aggressive settings, e.g. less beamforming, less noise cancelling, etc.; and/or
when physiological stress and moderate or low sound levels are detected: starting still another sound program.

Further aspects of the invention relate to a computer program for adjusting a hearing device, which, when being executed by a processor of the computer of the hearing device, carries out the steps of the method as described above and below, as well as to a computer-readable medium in which such a computer program is stored.

The computer-readable medium may be a memory of the computer of the hearing device. In general, a computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet, which allows downloading a program code. The computer-readable medium may be a non-transitory or transitory medium.

A further aspect of the invention relates to a hearing device adapted for carrying out the method as described above and below.

According to an embodiment of the invention, the hearing device comprises a sensor adapted for measuring at least one physiological indicator for a stress response of the user.

According to an embodiment of the invention, the sensor is at least one of a photoplethysmography sensor, an electrocardiography sensor, an electroencephalography sensor, an electroglottography sensor and an acceleration sensor.

A further aspect of the invention relates to a hearing system comprising the hearing device and a mobile device carried by the user of the hearing device and comprising a sensor adapted for measuring at least one physiological indicator for a stress response of the user, wherein the hearing device and the mobile device are interconnected over a wireless data connection. In other words, the sensor data used for determining the stress situation classification values may at least partially be generated by the mobile device.

It has to be understood that features of the method as described above and below may be features of the computer program, the computer-readable medium, the hearing device and the hearing system as described above and below, and vice versa.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig. 1 schematically shows a hearing system according to an embodiment of the invention.
Fig. 2 schematically shows a functional diagram of a hearing system illustrating a method for adjusting a hearing device according to an embodiment of the invention.
Fig. 3 shows a flow diagram for a method for adjusting a hearing device according to an embodiment of the invention.

The reference symbols used in the drawings, and their meanings, are listed in summary form in the list of reference symbols. In principle, identical parts are provided with the same reference symbols in the figures.

### DETAILED DESCRIPTION OF EXEMPLARY EMBODIMENTS

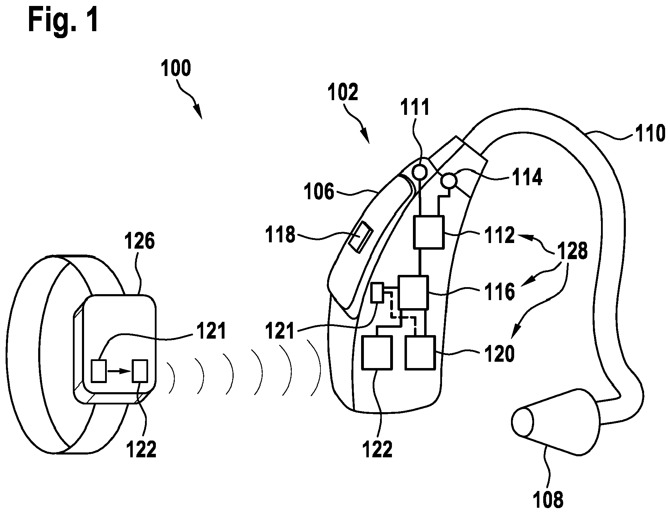

Fig. 1 schematically shows a hearing system 100 with a hearing device 102 in the form of a behind-the-ear device. It has to be noted that the hearing device 102 is a specific embodiment and that the method described herein also may be performed by other types of hearing devices, such as in-the-ear devices.

The hearing device 102 comprises a case 106 to be put behind an ear of a user, a dome tip 108 to be inserted into an external auditory canal of the ear, and a tube 110 connecting the case 106 with the dome tip 108. In the case 106, a microphone 111, a sound processor 112 and a sound output device 114, such as a loudspeaker (also called receiver), are provided. The case 106 may also contain a battery. The microphone 111 acquires environmental sound and generates audio data based on the acquired sound. The sound processor 112 amplifies the audio data. The sound output device 114 generates sound that is guided through the tube 110 and the dome tip 108 into the auditory canal of the user.

The hearing device 102 may further comprise a processor 116 adapted for adjusting sound processing parameters of the sound processor 112. For example, the sound processing parameters may adjust a frequency-dependent amplification of the audio data.

The sound processing parameters may be determined by a computer program run in the processor 116. The case 106 may have a button 118, for example a knob, to select a modifier, such as bass, treble, noise suppression, dynamic volume, etc., as well as levels and/or values of a selected modifier. These levels and/or values may then be applied to the audio data by the sound processor 112. Said functions may be implemented as computer programs stored in a memory 120 of the hearing device 102. The computer programs may be executed by the processor 116.

The hearing device 102 further has a sensor unit 121 comprising one or more sensors such as, for example, a photoplethysmography (PPG) sensor, an electrocardiography (ECG) sensor, an electroencephalography (EEG) sensor, an electroglottography (EGG) sensor and an acceleration sensor. One or more of the sensors may be placed in the auditory channel of the user. With the PPG and/or ECG sensor, a heart rate and/or heart rate variability of the user may be determined. With the EEG sensor, a frequency-dependent activity of the user's brain, such as an alpha-band activity, beta-band activity, gamma-band activity, etc., may be determined. The EGG sensor may be used for monitoring an activity of the user's voice and determining stress-related voice characteristics such as sound level, frequencies, e.g. a pitch frequency, speech rate, respiration rate, roughness, stuttering or trembling, i.e. tremolo or modulation. With the acceleration sensor, a movement of the user's head, which may be indicative for a physical activity of the user, may be determined. Sensor data generated by the sensor unit 121 may be processed by the processor 116. It is possible that the sensor data are temporarily stored in the memory 120.

The hearing device 102 may further comprise a sender/receiver 122 in wireless data communication with a sender/receiver 122 of a mobile device 126 carried by the user, which may be a smartphone, smartwatch or tablet computer. The mobile device 126 may comprise at least one of the above-mentioned sensors of the sensor unit 121 and send sensor data generated by these sensors to the hearing device 102.

The processor 116 analyzes the sensor data and, optionally, the audio data and determines a specific stress situation the user of the hearing device 102 is currently in. Depending on the stress situation, the processor 116 executes a specific sound program for adjusting the sound processing parameters accordingly, which will be described below in more detail. For example, beam forming and/or noise cancelation may be activated or intensified with the sound processor 112 when a stress situation related to high cognitive load is detected.

The processor 116, the sound processor 112 and the memory 120 may be seen as components of a computer 128 integrated in the hearing device 102.

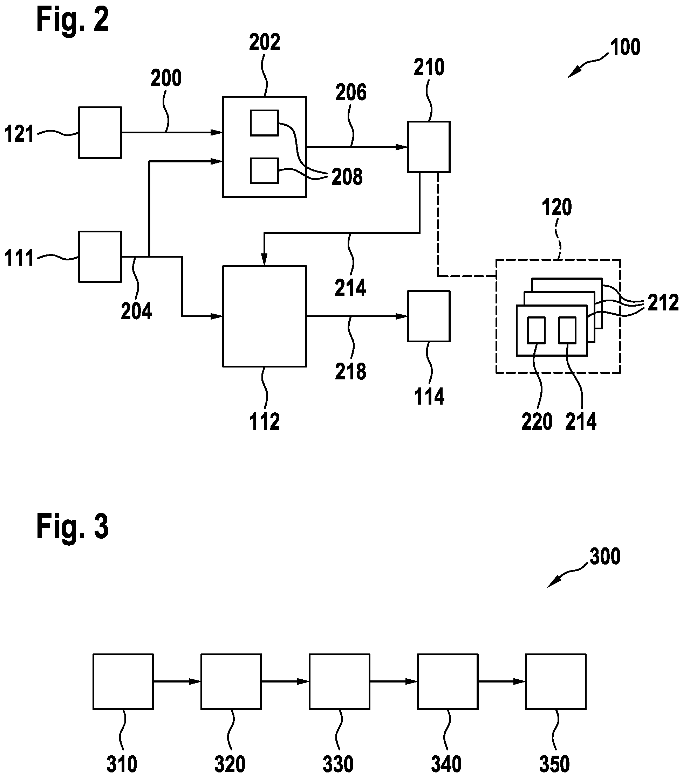

Fig. 2 shows a functional diagram of the hearing system 100. Also a method for adjusting the hearing device 102 will be illustrated with respect to Fig. 2.

The one or more sensors forming the sensor unit 121 of the hearing device 102 generate sensor data 200, which are supplied to a stress situation detector 202. If the hearing device 102 is coupled with a mobile device 126 provided with such sensors, the sensor data 200 also may be at least partially supplied by the mobile device 126. The microphone 111 of the hearing device 102 generates audio data 204. The audio data 204 may also may be sent from the mobile device 126 to the hearing device 102. In this case, the audio data 204 may be generated by a microphone of the mobile device 126 and/or may be received via the Internet and/or via a mobile telephone network during a telephone call.

Additionally to the sensor data 200, the audio data 204 may be supplied to the stress situation detector 202. The stress situation detector 202 analyzes the sensor data 202 and, optionally, the audio data 204 and determines stress situation classification values 206. The stress situation detector 202 may be implemented as a software module in the hearing device 102 and/or may be executed by the processor 116.

The stress situation classification values 206 are indicative of a specific stress situation of the user. For example, the stress situation classification values 206 may be probability values for different causes of stress, such as physical activity, sustained (high) workload, (high) cognitive load, emotional agitation, etc., and/or for specific stress-related features, such as, for example, heart rate variability, beta-band activity, gamma-band activity, acceleration, audio level, signal-to-noise ratio and/or voice characteristics as mentioned above. The audio data 204 may also be analyzed to determine whether the user is listening to media content, such as music, a movie, an audio book, etc., or not.

The stress situation classification values 206 may be determined with a stress classifier 208, i.e. a computer program adapted for identifying a specific stress situation by classifying the sensor data 200 and, optionally, the audio data 204. The stress situation detector 202 may also comprise a plurality of stress classifiers 208. Each stress classifier 208 may output at least one stress situation classification value 206 with respect to a specific stress situation. At least one of the stress classifiers 208 may be a sound classifier for classifying the audio data 204. It may be that at least one of the stress classifiers 208 is based on a machine learning algorithm, such as, for example, an artificial neuronal network, and/or has been trained with annotated sensor data and/or audio data.

The stress situation classification values 206 are input into a program selector 210, which, when specific criteria are met for a stress situation, starts a corresponding sound program. The program selector 210 may be implemented as a software module in the hearing device 102 and/or may be executed by the processor 116.

A sound program 212 is started by applying sound processing parameters 214 to the sound processor 112. The sound processor 112 processes the audio data 204 with the actual applied sound processing parameters 214. For example, the sound processor 112 is adapted for at least one of frequency-dependent amplifying the audio data 204, frequency-shifting the audio data 204 and frequency-compressing the audio data 204 in dependency of the applied sound processing parameters 214. The amplification and/or frequency shifting may be encoded with the sound processing parameters 214.

The processed audio data 218 are output with the sound output device 114, such as a loudspeaker, to the user.

The sound program 212 is stored in the memory 120 of the hearing device 102. The sound program 212 may comprise a data structure encoding in which sound situation the sound program 212 is started and which sound processing parameters 214 are applied to the sound processor 112 in this case. In general, the hearing device 102 may comprise at least two preset sound programs 212 with different sound processing parameters 214 associated with different situations of the user. For example, a first sound program 212 may define sound processing parameters 214 for a normal situation, characterized by an average stress level of the user, and a second sound program 212 may define sound processing parameters 214 for a stress situation, characterized by an above average stress level of the user, in particular a situation where the user is experiencing high cognitive load. It is also possible that the hearing device 102 comprises two or more different sound programs for different kinds of stress situations.

The sound program 212 is started when a specific set of stress situation classification values 206 is determined by the stress situation detector 202.

Each sound program 212 may comprise stress situation definition data 220 defining ranges for stress situation classification values 206. When the stress situation classification values 206 are within these ranges, it is assumed that the stress situation is met. Thus, the corresponding sound program 212 is started.

Furthermore, each sound program 212 may comprise preset sound processing parameters 214, which are applied to the sound processor 112 when the sound program 212 is started.

In general, a stress situation may be determined when a level of a naturally occurring beat-to-beat timing variation of the user's heartbeat is lower than a baseline in a rested state of the user. Usually, at a heart rate of about 60 bpm, individual heartbeats are between 0.9 and 1.1 seconds apart from each other, also called R-R intervals (RRi). This heart rate variability decreases with increasing physiological stress and may be measured in terms of a standard deviation with a PPG sensor or ECG sensor.

An EEG sensor may be used to measure general levels of the user's brain activity in terms of frequencies of electrical waves. Generally, higher frequencies refer to higher levels of brain activity. For example, a higher gamma-band activity may indicate that the user is learning, whereas a higher alpha-band activity may indicate that the user is sleeping.

Physical activity, in particular training, may also provoke physiological stress, i.e. decrease heart rate variability. Physical activity, such as sitting, walking, running, etc., may be measured with an accelerometer and/or a gyroscope.

Another potential cause for physiological stress are strong emotions as they may occur during an emotional conversation, while listening to music or an audio book, watching a movie, etc. Such emotional stress may be detected in the voice of the user, i.e. from an elevated sound level, increased pitch frequencies, faster speech and/or shorter speech pauses, stuttering, etc. Another indicator that something other than an acoustically difficult situation causes physiological stress to the user may be an existing connection for streaming media content from the mobile device 126 to the hearing device 102.

It is thus likely that an acoustically difficult situation is present when the user is experiencing physiological stress mainly caused by cognitive load rather than by physical activities, strong emotions, sustained workload, etc. In this case, the signal processing of the hearing device 102 may be adapted in an appropriate way as a countermeasure.

For example, it is likely that a heart rate variability lower than the user's individual baseline is due to a high cognitive load, i.e. difficult acoustic conditions, when a combination of a high sound pressure level, a low signal-to-noise ratio, an above average beta-band activity and an above average gamma-band activity is detected, while no relevant physical activity is detected, no media content is streamed and no emotional agitation is detected in the user's voice (or the user is not talking at all). On the other hand, the probability for a stress situation with high cognitive load may decrease in the case of recent physical activity, emotional agitation or high sustained workload.

For example, a high sound pressure level may be greater than 60 dB and a low signal-to-noise ratio may be less than +5 dB.

The sensor data 200 may be used to determine a long-term average and a short-term average for the beta-band activity and/or the gamma-band activity. An above average beta-band activity or above average gamma-band activity may be determined when the respective short-term average is at least 1.5 times higher than the respective long-term average.

To detect emotional agitation, baseline levels with respect to one or more characteristics of the user's voice may be measured, for example, when the user is talking without experiencing any relevant cognitive load. The baseline levels may then be used as reference values for further measurements of the voice characteristics.

It is also possible that the stress classifier 208 identifies, based on the audio data 204, whether the user is in a communication situation or not.

Fig. 3 shows a flow diagram for a method 300 for adjusting a hearing device. The method 300 may be performed by the hearing device 102 or the hearing system 100 as shown in Fig. 1, i.e. the hearing device 102 and the mobile device 126.

In step 310, the sensor data 200 from the sensor 121 and the audio data 204 from the microphone 111 are received at the computer 128.

In step 320, the stress situation detector 202, by means of the stress classifier(s) 208, analyzes the sensor data 200 and determines at least one stress situation classification value 206 with respect to a specific stress situation of the user. Optionally, the audio data 204 may be used by the stress situation detector 202 to determine the stress situation classification value 206.

In step 330, the sound program 212 is started when the stress classifier 208 determines at least one specific stress situation classification value 206. The sound program 212 comprises specific sound processing parameters 214 associated with the determined stress situation classification value 206.

In step 340, the sound processor 112 processes the audio data 204 based on the sound processing parameters 214, which are applied to the sound processor 112 when the sound program 212 is started.

In step 350, the sound output device 114 outputs the processed audio data 218 to the user.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. A single processor or controller or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- 100: hearing system
- 102: hearing device
- 106: case
- 108: dome tip
- 110: tube
- 111: microphone
- 112: sound processor
- 114: sound output device
- 116: processor
- 118: button
- 120: memory
- 121: sensor unit
- 122: sender/receiver
- 126: mobile device
- 128: computer
- 200: sensor data
- 202: stress situation detector
- 204: audio data
- 206: stress situation classification value
- 208: stress classifier
- 210: program selector
- 212: sound program
- 214: sound processing parameters
- 218: processed audio data
- 220: stress situation definition data
- 300: method for adjusting a hearing device
- 310: step of receiving sensor data and audio data
- 320: step of determining at least one stress situation classification value
- 330: step of starting a sound program
- 340: step of processing audio data
- 350: step of outputting processed audio data

## Claims

1. A method (300) for adjusting a hearing device (102), the hearing device (102) comprising a microphone (111) for generating audio data (204), a computer (128) with a sound processor (112) for processing the audio data (204) and a sound output device (114) for outputting the processed audio data (218) to a user of the hearing device (102), the method (300) comprising:
receiving (310) sensor data (200) at the computer (128) from a sensor (121) adapted for measuring at least one physiological indicator for a stress response of the user;
receiving (310) audio data (204) at the computer (128) from the microphone (111);
determining (320) at least one stress situation classification value (206) with respect to a specific stress situation of the user with a stress classifier (208) based on the sensor data (200);
when the stress classifier (208) determines at least one specific stress situation classification value (206): starting (330) a sound program (212) comprising sound processing parameters (214) associated with the at least one specific stress situation classification value (206);
processing (340) the audio data (204) with the sound processor (112), wherein the sound processing parameters (214) are applied to the sound processor (112) when the sound program (212) is started; and
outputting (350) the processed audio data (218) with the sound output device (114) to the user.

2. The method (300) of claim 1,
wherein the computer (128) comprises a plurality of stress classifiers (208), each of which outputting at least one stress situation classification value (206) with respect to a specific stress situation of the user;
wherein the sound program (212) comprises sound processing parameters (214) associated with a specific set of stress situation classification values (206);
wherein the sound program (212) is started when the stress classifiers (208) determine the specific set of stress situation classification values (206).

3. The method (300) of claim 2,
wherein at least one of the stress classifiers (208) is a sound classifier for additionally classifying the audio data (204).

4. The method (300) of one of the previous claims,
wherein the sensor data (200) comprise at least one of heart activity data acquired with a heart activity sensor, brain activity data acquired with a brain activity sensor, voice activity data acquired with a voice activity sensor and movement data acquired with a movement sensor;
wherein the stress classifier (208) determines the at least one stress situation classification value (206) by classifying at least one of the heart activity data, the brain activity data, the voice activity data and the movement data.

5. The method (300) of one of the previous claims, further comprising:
determining at least one reference value by analyzing sensor data (200) generated by the sensor (121) for a predefined period of time and/or when the user is in a specific situation;
wherein the stress classifier (208) determines the at least one stress situation classification value (206) additionally based on the at least one reference value.

6. The method (300) of one of the previous claims,
wherein the stress classifier (208) is based on a machine learning algorithm.

7. The method (300) of one of the previous claims,
wherein the sound program (212) comprises sound processing parameters (214) for adjusting at least one of a beamformer, a noise filter and an amplification of the sound processor (112).

8. The method (300) of one of the previous claims,
wherein the computer (128) comprises a plurality of sound programs (212), each of which comprising sound processing parameters (214) associated with at least one specific stress situation classification value (206).

9. A computer program comprising instructions which, when the computer program is executed by a computer (128), cause the computer (128) to carry out the method (300) of one of the previous claims.

10. A computer-readable medium having stored thereon the computer program of claim 9.

11. A hearing device (102), comprising:
a microphone (111) for generating audio data (204);
a computer (128) with a sound processor (112) for processing the audio data (204);
a sound output device (114) for outputting the processed audio data (218) to a user of the hearing device (102);
wherein the hearing device (102) is adapted for carrying out the method (300) of one of claims 1 to 8.

12. The hearing device (102) of claim 11, further comprising:
a sensor (121) adapted for measuring at least one physiological indicator for a stress response of the user.

13. The hearing device (102) of claim 12,
wherein the sensor (121) is at least one of a photoplethysmography sensor, an electrocardiography sensor, an electroencephalography sensor, an electroglottography sensor and a movement sensor.

14. A hearing system (100), comprising:
the hearing device (102) of one of claims 11 to 13;
a mobile device (126) carried by a user of the hearing device (102) and comprising a sensor (121) adapted for measuring at least one physiological indicator for a stress response of the user;
wherein the hearing device (102) and the mobile device (126) are interconnected over a wireless data connection.
